# EUROPÄISCHE PATENTSCHRIFT

(11) **EP 1 727 987 B1**
(45) Veröffentlichungstag und Bekanntmachung des Hinweises auf die Patenterteilung: **21.12.2016**
(21) Anmeldenummer: 05729129.6
(22) Anmeldetag: 17.03.2005
(51) Int. Cl.: F04D 29/04, F04D 13/06

(54) **PUMPE**
PUMP
POMPE

(30) Priorität: 18.03.2004 DE 102004013744; 20.04.2004 DE 102004019718
(43) Veröffentlichungstag der Anmeldung: 06.12.2006
(73) Patentinhaber: MEDOS Medizintechnik AG, 52222 Stolberg (DE)
(72) Erfinder: REUL, Helmut, verstorben (DE); AKDIS, Mustafa, 52074 Aachen (DE); MARSEILLE, Oliver, 55066 Aachen (DE)
(74) Vertreter: Castell, Klaus
(86) Internationale Anmeldenummer: PCT/DE2005/000508
(87) Internationale Veröffentlichungsnummer: WO 2005/090792

(56) Entgegenhaltungen:
- EP-A- 0 611 580
- EP-A- 1 013 294
- WO-A1-98/11650
- US-A- 3 771 910
- US-B1- 6 176 848

## Beschreibung

Die Erfindung betrifft eine Pumpe. Insbesondere betrifft die Erfindung eine Kreiselpumpe zur Förderung von Blut.

Aus dem Stand der Technik sind zahlreiche Pumpen mit Schaufelrädern bekannt. Beispielhaft für den medizintechnischen Bereich sei auf verschiedene Druckschriften hingewiesen:
So zeigt die US 6,116,862 eine Blutpumpe mit einem Flügelrad, welches in axialer Richtung kugelgelagert ist und in radialer Richtung durch einen sternförmigen Steg zentriert wird.
Die EP 0 904 117 B1 offenbart eine Blutpumpe, bei welcher ein Flügelrad axial ebenfalls über ein Kugellager fixiert ist. Die radiale Ausrichtung erfolgt über eine Hülse, welche über einen Steg mit einem Hauptgehäuse der Pumpe verbunden ist und dort als Gleitlager geführt wird.
In der DE 100 16 422 A1 und in der DE 101 08 810 A1 ist jeweils eine Blutpumpe offenbart, welche ein Schaufelrad in einem Gehäuse fest installiert hat.
Die DT 298 21 565 U1 zeigt eine lagerlose Blutpumpe. Ein Antrieb eines Schaufelrads erfolgt über eine Magnetkupplung. Das Schaufelrad ist in einem Pumpengehäuse innerhalb eines begrenzten Spiels frei beweglich und fördert bei Antrieb des Schaufelrads Blut in eine radiale Abströmrichtung. Um das axial zuströmende Blut radial abströmen zu lassen, übt das Schaufelrad einen Impuls auf das Blut aus. Damit das sich drehende Schaufelrad nicht infolge des Gegenimpulses axial am Gehäuse aufsetzt, weisen die Schaufeln des Schaufelrads an ihrer von der Zuströmung abgewandten Seite kleine Tragflächen auf.
In der EP 0 599 138 A2 ist ein Schaufelrad einer Blutpumpe auf einer in ein separates Gehäuse ragenden Welle angeordnet.
Die US 5,840,070 zeigt einen Rotor an einem Schaft, wobei sowohl an Schaufelrädern des Rotors als auch am Schaft eine Ausrichtung durch zahlreiche Magnete erfolgt.
Die DT 26 18 829 A1 zeigt eine mehrstufige Kreiselpumpe, bei welcher die einzelnen Pumpenstufen aus Kunststoff bestehen und durch axiale Vorspannung eine Verformung erfahren, bei welcher Seitenwände einzelner Stufengehäuse gegen ein Mantelrohr gepresst werden, um dort einen dichten Anschluss zu erreichen.
Die JP 2002-315824 A schlägt eine Blutpumpe vor, bei welcher ein Schaufelrad axial und radial berührungslos gelagert ist. Das Axiallager liegt stromabwärts des Schaufelrads und ist in Form zweier sphärischer Flächen gebildet, davon eine konkav und eine konvex ausgebildet. Eine dieser Oberflächen weist sich tangential verjüngende Fugen auf, sodass beim Drehen des Flügelrads Blut als Fluidpolster zwischen die sphärischen Flächen gepresst wird. Somit lagert sich das Flügelrad im Betrieb axial über ein Fluidpolster. Die radiale Stabilisierung erfolgt über Passivmagnete oder allein über ein Fluidpolster in einem dynamischen Druckschlitz. Dieser Druckschlitz liegt zwischen einem dem Flügelrad axial vorgelagerten Ring und dem Gehäuse. Der vorgelagerte Ring ist außenseitig mit einem Fischgrätmuster auf der Oberfläche versehen, damit die Stabilisierung erfolgt.
Die WO 98/11650 A1 zeigt Ausführungsbeispiele einer Blutpumpe mit einem vorteilhaften, jedoch recht komplexen Rotor-Stator-System derart, dass der Rotor berührungslos durch magnetische Kräfte innerhalb eines Gehäuses der Rotationspumpe gelagert und angetrieben werden kann. Der Hauptstrom durchfließt den Rotor mit seinen Schaufeln axial oder in einer anderen Ausführungsform zentrifugalbeschleunigt. Die Pumpe weist einen Sekundärkanal auf, wobei die Mündungsöffnung des Sekundärkanals zum Zuströmungskanal hin orientiert ist und in axialer Richtung vom Pumpengehäuse gegenüber der Hauptströmung verdeckt ist.

Der vorliegenden Erfindung liegt die Aufgabe zugrunde, eine kostengünstigere und verschleißärmere Pumpe zur Verfügung zu stellen.

Im Hinblick auf die mögliche Verwendung in der Medizintechnik als Blutpumpe betrifft die Erfindung zudem weitere Aspekte: Nach dem Stand der Technik, beispielsweise der bereits genannten US 6,116,862, ist es üblich, zur Lagerung des Schaufelrads zwei verschleißbehaftete mechanische Gleitlager zu verwenden, hiervon eines in Form einer Kugel-Kalotten-Lagerung und eines in Form einer Welle-Buchse-Lagerung. Die Kugel-Kalotten-Lagerung dient zur Aufnahme der Axiallasten auf das Schaufelrad, welche zunächst durch die Kupplungskräfte zwischen den Kupplungsmagneten des Motors und der Schaufelräder entstehen. Weiterhin wirken in axialer Richtung Strömungskräfte, die als hydraulische Druckkräfte während der Rotation des Schaufelrads entstehen. Die Kupplungsmagnete sind derart ausgelegt, dass für den gesamten Betriebsbereich der Pumpe die Kupplungskräfte stets so groß sind, dass das Schaufelrad eine resultierende Druckkraft auf die Kugel-Kalotten-Lagerung ausübt.

Die Gleitlagerung mit Welle und Buchse im Zuströmkanal der Pumpe bewirkt eine radiale Stabilisierung des Schaufelrads und dient einerseits zur Aufnahme radialer Strömungskräfte, welche beispielsweise bei unsymmetrischer Rotorlage entstehen können, sowie andererseits zur Aufnahme von Unwuchtkräften infolge von Fertigungstoleranzen oder Inhomogenitäten. Zudem fängt die Gleitlagerung etwaige aus der Kugel-Kalotten-Lagerung hervorgehende Kippkräfte auf.

Im Rahmen von in-vivo-Studien, bei denen derartige Blutpumpen erprobt wurden, zeigte sich immer wieder die Problematik der Blutgerinnselbildung im Zuströmbereich in die Pumpe. An den Stegen zur Fixierung der Buchse im Zentrum des Zuströmkanals kam es bei den Untersuchungen mit Blut zu Thrombenablagerungen. Im hinteren Kugel-Kalotten-Lager hingegen war aufgrund der starken Auswaschung dieses Lagers mittels einer durch Spülbohrungen erzwungenen Spülströmung keinerlei Thrombenablagerung zu beobachten. In bezug auf die medizintechnische Anwendung liegt der Erfindung die Aufgabe zugrunde, eine Blutpumpe zur Verfügung zu stellen, bei welcher eine Thrombenablagerung im Zuströmbereich der Pumpe erschwert wird.

Zur Lösung dieses Problems, schlägt die EP 1 013 294 A1 eine Blutpumpe vor, bei welcher die Gleitlagerung mit Welle und Buchse durch einen Magnetlager ersetzt wurde.

Weitere erfindungsgemäßen Aufgaben werden durch eine Pumpe, nämlich Kreiselpumpe zur Förderung von Blut, mit den Merkmalen des Anspruchs 1 gelöst. Weitere vorteilhafte Ausgestaltungen sind in den Unteransprüchen zu Anspruch 1 angegeben.

Die Pumpe gemäß Anspruch 1 kann sehr günstig produziert werden. Das Schaufelrad in der JP 2002-315824 A ist im Betrieb axial nicht körperhaft aufgelagert, sondern sitzt auf einem Druckfilm auf, den die sich tangential verengenden Fugen erzeugen. Die vorliegende Erfindung kann nach dem vorgestellten Aspekt auf solch aufwendige Maßnahmen optional verzichten. Sie hat nach diesem Aspekt erkannt, dass die Radiallagerung je nach Ausführungsform entscheidend sein kann.

Es sei erläutert, dass im Rahmen der vorliegenden Anmeldung eine strömungsmechanische Lagerung auch die Speziallagerfälle einer hydrodynamischen (also Reynolds-) Lagerung und einer sonstigen Fluidfilmlagerung als spezielle Ausführungsformen mit einschließen kann.

Ein strömungsmechanisches Radiallager ist zudem sehr kostengünstig auszuführen, weil die Zentrierung des Schaufelrads bei geeigneter Konstruktion des strömungsmechanischen Lagers selbsttätig erfolgen kann. Dies geschieht, wenn bei Auslenkung des Schaufelrads in demjenigen Bereich, in welchem sich das Schaufelrad einer Gehäusewand nähert, eine die Auslenkkraft übersteigende Rückstellkraft auf das Schaufelrad ausgeübt wird, bis dieses wieder zentriert im Zulaufkanal steht.

Die mechanische Lagerung am vom Zulauf abgewandeten Ende des Schaufelrads kann insbesondere eine Kugel-Kalotten-Lagerung oder eine sonstige Kipplagerung sein. Unter einer Kipplagerung wird eine Lagerung verstanden, bei welcher sich das Schaufelrad von seiner Hauptachse verschwenken kann, ohne dabei oder zumindest im wesentlichen ohne dabei an der körperhaften Axiallagerung radial auszuwandern.

Die Rückstellkraft kann als Zentrierungskraft insbesondere an einem Umfang des Schaufelrads ausgeübt werden, denn auf diese Weise ergeben sich vorteilhafte große Hebelarme, sodass auch eine kleine Rückstellkraft ausreichen kann, um ein recht großes, das Schaufelrad austreibendes Moment auszugleichen.

Nach einem weiteren Aspekt der Erfindung werden die Aufgaben durch eine Pumpe mit einem Schaufelrad und mit einem Gehäuse gelöst, wenn sich die Pumpe dadurch kennzeichnet, dass eine das Schaufelrad umgebende und an diesem fixierte Ringhülse innerhalb einer umgebenden Wandung des Gehäuses vorgesehen ist.

Eine an die Rotation des Schaufelrads gekoppelte Ringhülse, welche um das Schaufelrad herum verläuft, macht die Pumpe einer strömungsmechanischen vorderen Lagerung besonders zugänglich. Durch die Rotation der Ringhülse bei Rotation des Schaufelrads wird bereits die Wahrscheinlichkeit von Strömungstotbereichen an der Ringhülse weitestgehend eliminiert. Zudem kann ein strömungsmechanisches Lager besonders effektiv Rückstellkräfte am Umfang der Ringhülse ausüben.

Eine besonders kostengünstige Bauform, die zudem sehr kompakt ist, ermöglicht das Anordnen der Ringhülse um das Schaufelrad herum. Es sei darauf hingewiesen, dass sich die Ringhülse nicht notwendigerweise über die gesamte axiale Länge des Schaufelkörpers erstrecken muss. Sie sollte jedoch zumindest über das stromaufwärtige Viertel, besser über die stromaufwärtige Hälfte, des Schaufelkörpers herum angeordnet sein. Idealerweise ist der Schaufelkörper des Schaufelrads mit seiner stromaufwärtigen Spitze axial zumindest etwa bis auf die Höhe des stromaufwärtigen Beginns der Ringhülse vorgezogen. Ein solches Merkmal kennzeichnet eine sehr kompakte Bauform. Außerdem ist die Massenträgheit des Schaufelrads um die Kugel-Kalotten-Lagerung erhöht, was das Flügelrad zusätzlich gegen ein Auslenken stabilisiert.

Der Lagerring in der JP 2002-315824 A ist axial vollständig vor dem Schaufelkörper angeordnet, also im Betrieb stromaufwärts. Er umgibt das Schaufelrad nicht.

Nach einem weiteren Aspekt löst die Aufgaben auch eine Pumpe mit einem Schaufelrad mit einer Drehachse und mit einem diagonal-axialen Hauptströmungskanal durch das Schaufelrad und mit einem Zuströmkanal zum Hauptströmungskanal des Schaufelrads, wobei die Pumpe einen Sekundärkanal mit einer Speiseöffnung und einer Mündungsöffnung aufweist, von welchen die Mündungsöffnung zum Zuströmkanal orientiert ist.

Der Sekundärkanal erzwingt bei geeigneter Konstruktion eine Sekundärströmung in einer solchen Stärke, dass die Strömung durch den Sekundärkanal für die strömungsmechanische Lagerung des Schaufelrads verwendet werden kann. Die Lagerströmung kann beim Verlauf im Sekundärkanal von der Hauptströmung getrennt geführt werden und insbesondere auch der Hauptströmung durch das Schaufelrad im wesentlichen entgegengesetzt orientiert verlaufen. Dies ermöglicht eine automatische Zirkulation von Fluid durch das strömungsmechanische Lager, ohne dass von der Hauptströmung eine Abzweigung erfolgen muss.

Es ist von Vorteil, wenn eine Gehäusewandung im Bereich der Speiseöffnung einen kontinuierlichen Oberflächenverlauf aufweist. Jegliche sprunghafte Stelle an der Kanalwand nimmt der an der Speiseöffnung beginnenden Sekundärströmung Energie und mindert somit die radiale Stabilität des Schaufelrads. In der JP 2002-315 824 A weist das Gehäuse demgegenüber an einer Speiseöffnung zum Ringspalt eine umlaufende Innennut auf.

Um die Energie der Abströmung vom Flügelrad bestmöglich zu nutzen, wird vorgeschlagen, dass die Speiseöffnung radial weiter außen angeordnet ist als der Schaufelkörper reicht. Bei einer solchen Bauform ist sichergestellt, dass die Sekundärströmung erst dort Blut abzweigt, wo dieses vollständig von den Schaufeln des Schaufelrads abgeströmt ist. Es hat somit die höchste Energie im gesamten Bereich der Pumpe.

Es ist ersichtlich, dass unter dem gleichen Aspekt die Speiseöffnung des Sekundärkanals stromabwärts einer Mündung einer Spülströmung angeordnet sein sollte, wenn eine solche vorgesehen ist und die Spülströmung zurück in die Hauptströmung führt.

Der Sekundärkanal kann insbesondere im wesentlichen zylindermantelförmig, im wesentlichen kegelstumpfmantelförmig oder im wesentlichen aus solchen Formen zusammengesetzt sein. Hierzu ist es konstruktiv von Vorteil, wenn eine Ringhülse und eine diese umgebende Gehäusewandung über eine gemeinsame Verschwenkung hinweg parallel zueinander verlaufen. Axial abwärts der Verschwenkung können die beiden Bauteile separat, insbesondere aber gemeinsam, vor allem einen Winkel zur Drehachse des Schaufelrads von etwa 20 bis 90 ° einnehmen.

Es wird vorgeschlagen, dass im Betrieb der Pumpe die Lagerströmung von einer Abströmung vom Schaufelrad gespeist wird. Unmittelbar beim Verlassen des Schaufelrads ist die Energiehöhe des gepumpten Fluids besonders hoch, so dass gegenüber einem stromaufwärtigen Punkt eine Druckdifferenz herrscht, welche zum Erzeugen der Sekundärströmung ausgenutzt werden kann.

Um das Energiegefälle bestmöglich auszunutzen, kann die Sekundärströmung in die Zuströmung zum Schaufelrad münden. Unmittelbar vor dem Schaufelrad ist die Energiehöhe des gepumpten Fluids am niedrigsten.

Es sei darauf hingewiesen, dass eine Pumpe mit einem Schaufelrad, insbesondere eine derartige Blutpumpe, mit einer Lagerströmung, welche im Betrieb einer Hauptströmung im wesentlichen entgegengerichtet verläuft, auch unabhängig von sämtlichen übrigen Merkmalen vorliegender Erfindung vorteilhaft und erfinderisch ist. Gleiches gilt für eine Pumpe, insbesondere eine Blutpumpe, bei welcher im Betrieb eine Lagerströmung von einer Abströmung vom Schaufelrad gespeist wird, sowie für eine Pumpe, insbesondere eine Blutpumpe, bei welcher im Betrieb eine Lagerströmung in eine Zuströmung zum Schaufelrad mündet.

In einer bevorzugten Ausführungsform ist ein Sekundärkanal flächig erstreckt und in einer Dimension senkrecht zu seiner Fläche mehr als 100 µm, bevorzugt etwa 300 bis 700 µm, besonders bevorzugt etwa 500 µm, breit. Klassische hydrodynamische Gleitlager fangen die auf einen Rotor wirkenden Kräfte durch eine in einem hydrodynamischen Schmierfilm entstehende Reynolds-Druckkraft auf. Hierzu muss die Dicke des Schmierfilms derart klein sein, dass die viskosen Kräfte die Trägheitskräfte übertreffen. Übliche Spaltbreiten liegen bei etwa 10 µm. Nur dann können die im Viskoseschmierfilm entstehenden Kräfte den auf den Rotor wirkenden äußeren Kräften das Gleichgewicht halten und das Aufsetzten des rotierenden Lagerteils auf das stationäre Lagerelement verhindern. Solange eine Mischreibung verhindert wird, bleibt diese Lagerung verschließfrei. Der wesentliche Nachteil einer solchen hydrodynamischen Lagerung für die Anwendung in einer Blutpumpe ist jedoch, dass die geringe Breite des Schmierspaltes die Hämolyserate der Blutpumpe signifikant erhöht und somit einen blutschonenden Einsatz am Patienten einschränkt. Darüber hinaus liegt bei dermaßen kleinen Spaltbreiten auch eine erhöhte Gefahr von Strömungstotbereichen vor, so dass sogar die Gefahr der Trombenablagerung im Lagerbereich erhöht wird, was bei Blutpumpen für den Langzeiteinsatz unbedingt zu vermeiden ist. Gemäß einem Aspekt der vorliegenden Erfindung kann Blut jedoch für eine strömungsmechanische Lagerung auch bei deutlich größeren Spaltbreiten, insbesondere bei Spaltbreiten bis etwa 500 µm, genutzt werden. Dies senkt die Gefahren der Hämolyse und der Thrombogenität erheblich.

Bei aufwendigen Versuchen zur Strömungsführung hat sich zudem gezeigt, dass es von Vorteil ist, wenn im Betrieb die Lagerströmung überwiegend axial in einem tangential und axial erstreckten Sekundärkanal verläuft. Der Sekundärkanal kann insbesondere ein Ringspalt sein.

Es sei ausdrücklich darauf hingewiesen, dass eine Blutpumpe mit einer strömungsmechanischen Lagerströmung, welche überwiegend axial in einem tangential und axial erstreckten Sekundärkanal verläuft, auch für sich genommen vorteilhaft und erfinderisch ist, insbesondere wenn der Sekundärkanal ein Ringspalt um das Schaufelrad ist. Gleiches gilt für eine Blutpumpe mit einem Sekundärkanal für eine strömungsmechanische Lagerung, wobei der Sekundärkanal eine Spaltbreite von mehr als 100 µm, bevorzugt von etwa 300 bis 700 µm, besonders bevorzugt von etwa 500 µm, hat.

Alternativ und kumulativ zum Vorgenannten wird vorgeschlagen, dass die Lagerströmung im Betrieb zwischen 5 und 50 %, bevorzugt zwischen 10 und 50 %, besonders bevorzugt zwischen 20 und 50 %, vor allem etwa 30 %, der Hauptströmung ausmacht. Es hat sich gezeigt, dass bei einer Strömungseinstellung in diesem Spektrum ein ausreichendes strömungsmechanisches Polster für das Schaufelrad erzeugt wird und Strömungsstagnation im Sekundärkanal sicher vermieden wird. Für eine Blutpumpe eignen sich besonders Volumenströme der Hauptströmung zwischen etwa 2 bis 8 l/min, bevorzugt von etwa 3 bis 7 l/min, besonders bevorzugt von etwa 5 l/min. Eine Sekundärströmung kann demzufolge insbesondere zwischen 0,1 und 4 l/min, bevorzugt zwischen etwa 0,2 und 41/min, besonders bevorzugt zwischen etwa 0,4 und 41/min, vor allem bei etwa 1,5 l/min liegen.

Es wird vorgeschlagen, dass der Ringspalt so ausgeführt ist, dass bei zentriertem Schaufelrad keine Berührung zwischen dem Schaufelrad und einer umgebenden Wandung des Pumpengehäuses auftritt.

Erfindungsgemäß ist die Mündungsöffnung des Sekundärkanals in axialer Richtung durch einen radialen Vorsprung vom Pumpengehäuse verdeckt.

Vorteil eines umlaufenden Ringspalts um das Schaufelrad ist eine verschleißfreie Lagerung, wobei ein Vorsprung des Gehäuses die Mündungsöffnung des Sekundärkanals genau so abdecken kann, dass trotz der umlaufenden Öffnung die Hauptströmung nicht in unkontrollierbarer Weise durch den Sekundärkanal strömt. Zusätzlich zum strömungsmechanischen Lager kann eine mechanische Spielbegrenzung für das Schaufelrad vorgesehen sein, wobei insbesondere bei etwa 2° Auslenkung des Schaufelrads von der Drehachse eine weitere Auslenkung mechanisch begrenzt sein kann.

Für den Einsatz in Blutpumpen eignet sich besonders ein Sekundärkanal mit einer axialen Länge von etwa 1 bis 10 mm, bevorzugt von 2 bis 8 mm, besonders bevorzugt von etwa 5 mm. Für die Dimension des Schaufelrads wird ein Durchmesser von 5 bis 40 mm, bevorzugt von 5 bis 30 mm, besonders bevorzugt von 15 bis 25 mm, vor allem von etwa 20 mm, vorgeschlagen. Der Antrieb des Schaufelrads kann vorteilhaft über eine berührungslose Antriebskupplung zu einem Motor erfolgen, insbesondere über eine Kupplung rotierender Magnete. Wenn die Magnete axial magnetisiert sind, können größere Antriebsmomente vom Motor auf das Schaufelrad übertragen werden.

Es wird vorgeschlagen, dass eine Einrichtung zum Erhöhen der Strömungsenergie durch einen Hauptströmungskanal der Pumpe strömenden Fluids zwischen einer Speiseöffnung und einer Mündungsöffnung des Sekundärkanals vorgesehen ist. Auf diese Weise kann ein deutliches Druckgefälle zwischen Speisung und Mündung des Sekundärkanals erzeugt werden, was die Strömungsrichtung des Fluids im Sekundärkanal sicher vorhersagbar macht. Die genannten und weitere Aspekte der Erfindung werden nachfolgend anhand eines Ausführungsbeispiels unter Bezugnahme auf die Zeichnung weiter erläutert. In der Zeichnung zeigen
- Figur 1: einen schematischen Längsschnitt durch eine Blutpumpe mit einem erfindungsgemäß gelagerten Schaufelrad,
- Figur 2: eine schematische Ansicht der Blutpumpe aus Figur 1 gemäß dortiger Kennzeichnung und
- Figur 3: eine ausschnittsweise Vergrößerung des Längsschnitts aus Figur 1 mit angedeuteten Strömungsbahnen.

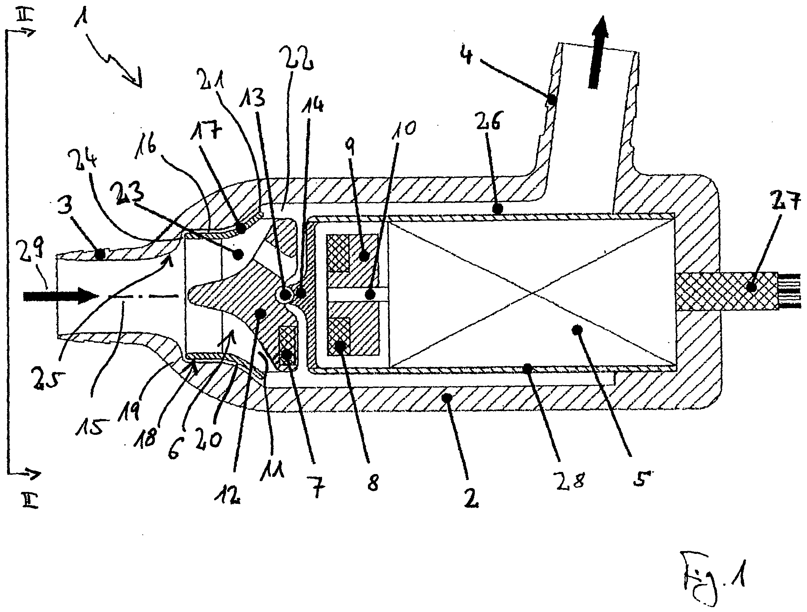

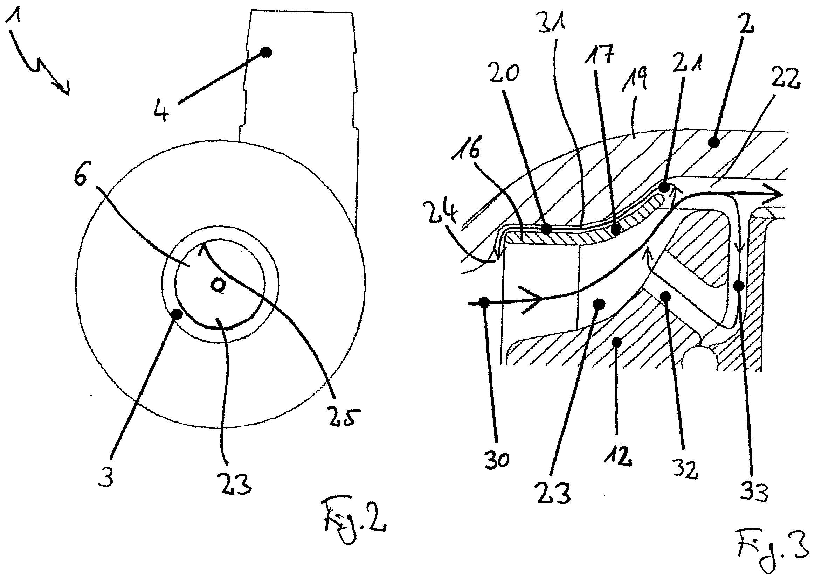

Die Blutpumpe 1 in den Figuren 1, 2 und 3 besteht im wesentlichen aus einem Pumpengehäuse 2 mit einem Pumpeneinlassstutzen 3 und einem Pumpenauslassstutzen 4. Innerhalb des Gehäuses 2 sind ein Motor 5 und ein Schaufelrad 6 angeordnet, wobei eine Rotationskraft vom Motor 5 auf das Schaufelrad 6 über eine Magnetkupplung 7, 8 bewirkt wird. Seitens des Motors sind die Magnete 8 an einem Polschuh 9 auf einer Rotorwelle 10 fixiert.

Seitens des Schaufelrads 6 sind die Magnete 7 unmittelbar in den Boden eines Schaufeln 11 tragenden zentralen Schaufelkörpers 12 des Schaufelrads 6 integriert. Der zentrale Schaufelkörper 12 ist über eine Kugel-Kalotten-Lagerung 13, 14 in axialer Richtung bezüglich einer Drehachse 15 des Schaufelrads 6 aufgelagert. Die Schaufeln 11 können insbesondere in sich gerade und auf dem zentralen Schaufelkörper 12 radial angeordnet sein oder beispielsweise schneckenförmig oder anderweitig geschwungen sein.

Der zentrale Schaufelkörper 12 des Schaufelrads 6 ist mit einer im wesentlichen in einem vorderen Abschnitt 16 zylindermantelförmigen und in einem hinteren Abschnitt 17 kegelstumpfmantelförmigen Hülse 18 fest verbunden. Die Hülse 18 liegt mit einem geringfügigen Spiel in einer Wandung 19 des Gehäuses 2 der Blutpumpe 1, wobei ein Ringspalt 20 zwischen der Wandung 19 und der Hülse 18 bei zentriertem Flügelrad 6 verbleibt. Der Ringspalt 20 hat in seinem axialen Verlauf eine im wesentlichen konstante Spaltbreite von etwa 500 µm.

Eine Speiseöffnung 21 des Ringspalts 20 liegt unmittelbar an einem stromabwärtigen Ende 22 des Flügelrads 6 bezüglich eines Hauptstromkanals 23. Eine stromzugewandte Mündungsöffnung 24 des Ringspalts 20 ist von einem Vorsprung 25 der Wandung 19 des Gehäuses 2 radial verdeckt. Am stromabwärtigen Ende 22 des Flügelrads 6 und an der Speiseöffnung 21 des Ringspalts 20 schließt sich ein ringförmiger Durchflusskanal 26 zum Transport von Blut durch die Blutpumpe 1 an. Dieser mündet in den Auslaufstutzen 4.

Der Radius der Schaufeln 11 des Schaufelrads 6 beträgt etwa 10 mm, die Länge des Ringspalts beträgt etwa 5 mm.

Im Betrieb der Blutpumpe 1 wird der Motor 5 von einer Stromzufuhr 27 gespeist und überträgt eine Rotation der Welle 10 des Schaufelrads 6 mit etwa 6000 U/min über die Magnetkupplung 7, 8 auf das Flügelrad 6. Dabei wird der Motor 5 von einem Motorgehäuse 28 innerhalb des Pumpengehäuses 2 gegen das in die Pumpe eintretende und ihn umströmende Blut geschützt. Übliche Umdrehungszahlen können für eine Blutpumpe insbesondere zwischen 3000 und 10000 U/min, insbesondere bei etwa 5000 U/min, liegen.

Das Blut wird von dem rotierenden Flügelrad 6 in einer Zuströmungsrichtung 29 in die Blutpumpe 1 eingesaugt und fließt in einer Hauptströmung 30 durch das Flügelrad 6 über dessen Schaufeln hindurch. Innerhalb des Hauptkanals 23 wird das Blut durch die Schaufeln des Schaufelkörpers 12 beschleunigt und gelangt so zum stromabwärtigen Ende 22 des Schaufelrades 6. Dort fließt es überwiegend in den breiten ringförmigen Hauptkanal 26 zum Abflussstutzen 4.

Gleichzeitig stellt sich im Ringspalt 20 eine axial gerichtete Lagerströmung 31 zwischen der Hülse 16, 17 und der Wandung 19 des Gehäuses 2 ein. Da die Hülse 18 mit dem zentralen Schaufelkörper 12 des Schaufelrads 6 fest verbunden ist, entsteht neben der axial gerichteten Lagerströmung 31 auch eine Rotationsströmung im Ringspalt 20. Wegen der großen Spaltbreite leistet diese allerdings keinen nennenswerten Beitrag zur radialen Stabilisierung des Schaufelrads 6.

Das durch den Ringspalt 20 im wesentlichen axial strömende Blut wirkt zusammen mit der Hülse 18 am Umfang des Schaufelrads 6 als ein strömungsmechanisches Lager. Bei zentraler Lage des Schaufelrads 6 liegt eine symmetrische Druckverteilung im Ringspalt 20 vor, so dass die resultierende Kraft in radialer Richtung ein Nullvektor ist. In dieser Position sind auch die seitens der Magnetkupplung 7, 8 auf das Schaufelrad 6 wirkenden Kippkräfte gleich Null. Sobald das Schaufelrad 6 gemäß dem ihm zur Verfügung stehenden Spiel eine Kippbewegung um das hintere Kugel-Kalotten-Lager 13, 14 ausführt, resultiert in Folge der unsymmetrischen Lage der Magnete 7, 8 eine Kippkraft in der Magnetkupplung, welche eine zusätzliche Auslenkkraft auf das Schaufelrad 6 in der ausgelenkten Richtung ausübt. Dieser instabile Auslenkeffekt wird durch die zusätzlich einsetzenden seitlichen Strömungskräfte auf die Schaufeln des zentralen Schaufelkörpers 12 noch verstärkt.

Durch die vorliegend vorgeschlagene strömungsmechanische Lagerung 31 werden seitliche Kräfte jedoch ausgeglichen und das Schaufelrad 6 wieder in eine zentrale Lage zurückgestellt, bevor die Hülse 18 an der Wandung 19 des Pumpengehäuses 2 anschlagen kann. Hierzu durchströmt der Lagerstrom 31 den Ringspalt 20 mit etwa 2 l/min, während der Hauptstrom 30 etwa 5 l/min beträgt.

Der relativ starke Lagerstrom wird durch die große Druckdifferenz zwischen den beiden Enden der Hülse, nämlich der Speiseöffnung 21 und der Mündungsöffnung 24, bewirkt, welche sich im Betrieb der Pumpe 1 einstellt. Das Schaufelrad 6 baut einen Druckgradienten im Blut in diagonal-axialer Richtung auf, so dass der Ringspalt 20 der Hauptströmung 30 entgegengesetzt in etwa in axialer Richtung durchströmt wird.

Mikroskopisch basiert der Rückstelleffekt des Schaufelrads 6 darauf, dass bei seitlicher Auslenkung des Schaufelrads 6 um die Kugel-Kalotten-Lagerung 13, 14 im Ringspalt 20 eine ungleichmäßige Spalthöhe entlang des Umfangs der Hülse 18 vorliegt. Infolgedessen herrscht im verengten Spaltbereich ein größerer Druck als auf der gegenüberliegenden Seite des Ringspalts 20. Das resultierende Ungleichgewicht bewirkt die zentrierende Rückstellkraft.

Unabhängig von der strömungsmechanischen Lagerung 31 sind im Schaufelkörper 12 des Schaufelrads 6 Spülbohrungen 32 vorgesehen, welche eine Spülströmung 33 induzieren, so dass an der körperhaften Lagerung 13, 14 des Schaufelrads 6 eine Thrombenbildung ebenfalls vermieden wird.

In der Wortwahl der vorliegenden Anmeldung fungiert die Kugel-Kalotten-Lagerung 13, 14 als körperhaftes Axiallager, während die Lagerströmung 31 mit der Wandung 19 und der Hülse 18 ein strömungsmechanisches Radiallager bildet. Das strömungsmechanische Radiallager übt seine Kraft auf die Außenseite der mit den Schaufeln 12 verbundenen Hülse 18 aus, somit am Umfang des Schaufelrads 6. Insofern dient der Ringspalt 20 als Sekundärkanal und trennt die Lagerströmung 31 von der Hauptströmung 30 durch das Schaufelrad 6.

Die Speiseöffnung 21 des Ringspalts 20 liegt am stromabwärtigen Ende 22 des Schaufelrads 6 und wird daher unmittelbar von einer Abströmung vom Schaufelrad 6 gespeist. Auf der stromzugewandten Seite mündet die Mündungsöffnung 24 der Lagerströmung 31 unmittelbar in die Zuströmung 29 zum Schaufelrad 6.

Da der Ringspalt 20 im wesentlichen zylindermantelförmig beziehungsweise kegelstumpfmantelförmig ist, erstreckt sich der Freiraum, welcher für die Lagerströmung zur Verfügung steht, bezüglich der Drehachse 15 tangential und axial, während die Lagerströmung überwiegend axial vorliegt.

## Patentansprüche

1. Pumpe (1), nämlich Kreiselpumpe zur Förderung von Blut, mit einem Schaufelrad (6) mit einer Drehachse (15) und mit einem Hauptströmungskanal (23) durch das Schaufelrad (6) und mit einem Zuströmkanal (29) zu dem Schaufelrad (6) in einem Gehäuse (2, 3), sowie mit einem Sekundärkanal (20) mit einer Speiseöffnung (21) und einer Mündungsöffnung (24), wobei die Mündungsöffnung (24) zum Zuströmkanal (29) orientiert ist, wobei:
- der Zuströmkanal normal zu der Drehachse in axialem Verlauf einen stetigen Querschnittsverlauf aufweist,
- der Hauptströmungskanal diagonal-axial ausgebildet ist, so dass im Betrieb eine Zuströmung im wesentlichen axial in das Schaufelrad läuft und eine Abströmung im wesentlichen diagonal aus dem Schaufelrad läuft, und dass
- das Schaufelrad in axialer Richtung körperhaft aufgelagert ist und von der Drehachse radial auslenkbar ist.
**dadurch gekennzeichnet, dass** die Mündungsöffnung (24) des Sekundärkanals durch einen radialen Vorsprung des Pumpengehäuses verdeckt ist.

2. Pumpe nach Anspruch 1, ***dadurch gekennzeichnet, dass*** der Querschnitt des Pumpengehäuses in dessen axialem Verlauf über das Schaufelrad ausschließlich konstant bleibt oder sich aufweitet.

3. Pumpe nach Anspruch 1 oder 2, ***dadurch gekennzeichnet, dass*** der Sekundärkanal ein berührungsloser Ringspalt zwischen dem Schaufelrad und der umgebenden Wandung des Pumpengehäuses bei zentriertem Schaufelrad ist, wobei eine Außenfläche des Schaufelrads bevorzugt glatt ausgebildet ist.

4. Pumpe nach einem der vorhergehenden Ansprüche, ***dadurch gekennzeichnet, dass*** der Sekundärkanal flächig erstreckt ist und in einer Dimension senkrecht zu seiner Fläche eine Ausdehnung von mehr als 100 µm, bevorzugt von etwa 300 bis 700 µm, besonders bevorzugt von etwa 500 µm, hat.

5. Pumpe nach einem der vorhergehenden Ansprüche, ***gekennzeichnet durch*** eine das Schaufelrad umgebende und an diesem fixierte Ringhülse innerhalb der umgebenden Wandung des Gehäuses, wobei sich die Ringhülse bevorzugt axial zumindest über das stromaufwärtige Viertel der Länge eines Schaufelkörpers erstreckt.

6. Pumpe nach einem der vorhergehenden Ansprüche, ***dadurch gekennzeichnet, dass*** im Betrieb im Sekundärkanal eine von einer durch das Schaufelrad verlaufenden Hauptströmung getrennte Lagerströmung fließt.

7. Pumpe nach einem der vorhergehenden Ansprüche, ***dadurch gekennzeichnet, dass*** im Betrieb eine Lagerströmung einer Hauptströmung im Wesentlichen entgegengerichtet orientiert verläuft.

8. Pumpe nach einem der vorhergehenden Ansprüche, ***dadurch gekennzeichnet, dass*** im Betrieb eine Lagerströmung von einer Abströmung vom Schaufelrad gespeist wird, wobei bevorzugt die Speiseöffnung radial weiter außen angeordnet ist als der Schaufelkörper reicht.

9. Pumpe nach einem der vorhergehenden Ansprüche, ***dadurch gekennzeichnet, dass*** im Betrieb der Pumpe bei angetriebenem Schaufelrad ein strömungsmechanisches Radiallager eine Zentrierungskraft auf das Schaufelrad ausübt, sobald dieses ausgelenkt ist.

10. Pumpe nach einem der vorhergehenden Ansprüche, ***dadurch gekennzeichnet, dass*** ein Axiallager ein Kugel-Kalotten-Lager oder ein sonstiges Kipplager ist.

## Claims

1. A pump (1), specifically a centrifugal pump for conveying blood, with an impeller (6) with a rotation axis (15) and with a main flow channel (23) through the impeller (6) and with an inflow channel (29) to the impeller (6) in a housing (2, 3), and with a secondary channel (20) with a feed opening (21) and an outlet opening (24), the outlet opening (24) being oriented towards the inflow channel (29),
- the inflow channel having in axial progression a constant cross-sectional profile normal to the rotation axis,
- the main flow channel being diagonal-axial, so that inflow runs substantially axially into the impeller during operation and outflow runs substantially diagonally out of the impeller, and
- the impeller being physically supported in the axial direction and being radially deflectable from the rotation axis,
***characterised in that*** the outlet opening (24) of the secondary channel is covered by a radial projection of the pump housing.

2. The pump according to Claim 1, ***characterised in that*** the cross section of the pump housing in the axial progression thereof only remains constant or widens over the impeller.

3. The pump according to Claim 1 or 2, ***characterised in that*** the secondary channel is a contactless annular gap between the impeller and the surrounding wall of the pump housing when the impeller is centred, wherein an outer face of the impeller is preferably smooth.

4. The pump according to any one of the preceding claims, ***characterised in that*** the secondary channel is extended in a planar manner and, in a dimension perpendicular to the face thereof, has an expansion of more than 100 µm, preferably of approximately 300 to 700 µm, particularly preferably of approximately 500 µm.

5. The pump according to any one of the preceding claims, ***characterised by*** an annular sleeve, which surrounds the impeller and is fixed to same, inside the surrounding wall of the housing, wherein the annular sleeve preferably extends axially at least over the upstream quarter of the length of an impeller blade body.

6. The pump according to any one of the preceding claims, ***characterised in that*** a bearing flow, which is separate from a main flow running through the impeller, flows in the secondary channel during operation.

7. The pump according to any one of the preceding claims, ***characterised in that*** a bearing flow runs substantially in the opposite direction to a main flow during operation.

8. The pump according to any one of the preceding claims, ***characterised in that*** a bearing flow is fed by an outflow from the impeller during operation, wherein the feed opening is preferably arranged radially further outwards than the impeller blade body extends.

9. The pump according to any one of the preceding claims, ***characterised in that*** when the pump is operated with a driven impeller, a flow-mechanical radial bearing exerts a centring force on the impeller as soon as said impeller is deflected.

10. The pump according to any one of the preceding claims, ***characterised in that*** an axial bearing is a universal ball joint bearing or other tilting bearing.

## Revendications

1. Pompe (1), à savoir pompe centrifuge pour charrier du sang, comportant une roue à aube (6) dotée d'un axe de rotation (15) et d'un canal d'écoulement principal (23) dans la roue à aube (6) et un canal d'afflux (29) vers la roue à aube (6) dans un boîtier (2, 3), de même qu'un canal secondaire (20) doté d'un orifice d'alimentation (21) et d'un orifice d'embouchure (24), l'orifice d'embouchure (24) étant orienté en direction du canal d'afflux (29),
- le canal d'afflux présentant, à la perpendiculaire de l'axe de rotation, en extension axiale, une extension constante de section transversale,
- le canal d'écoulement principal étant réalisé avec un axe diagonal-axial, de sorte que, en cours de fonctionnement, un afflux rentre sensiblement axialement dans la roue à aube et un reflux sort sensiblement diagonalement de la roue à aube, et que
- la roue à aube s'appuie physiquement dans le sens axial et peut être déviée radialement de l'axe de rotation,
***caractérisée en ce que*** l'orifice d'embouchure (24) du canal secondaire est couvert par une saillie radiale du boîtier de la pompe.

2. Pompe selon la revendication 1, ***caractérisée en ce que*** la section transversale du boîtier de pompe reste exclusivement constante ou s'élargit dans son extension axiale au-delà de la roue à aube.

3. Pompe selon la revendication 1 ou 2, ***caractérisée en ce que*** le canal secondaire est un interstice annulaire sans contact entre la roue à aube et la paroi environnante du boîtier de pompe lorsque la roue à aube est centrée, une surface extérieure de la roue à aube ayant de préférence une conformation lisse.

4. Pompe selon une des revendications précédentes, ***caractérisée en ce que*** le canal secondaire s'étend superficiellement et a, dans une dimension perpendiculaire à sa surface, une extension de plus de 100 µm, préférentiellement d'environ 300 à 700 µm, plus préférentiellement d'environ 500 µm.

5. Pompe selon une des revendications précédentes, ***caractérisée par*** un manchon annulaire entourant la roue à aube et fixé à celle-ci dans la paroi environnante du boîtier, le manchon annulaire s'étendant de préférence axialement, du moins sur le quart dirigé vers l'amont de la longueur d'un corps d'aube.

6. Pompe selon une des revendications précédentes, ***caractérisée en ce que,*** en cours de fonctionnement, un flux de base séparé d'un flux principal par la roue à aube s'écoule dans le canal secondaire.

7. Pompe selon une des revendications précédentes, ***caractérisée en ce que**,* en cours de fonctionnement, un flux de base s'étend avec une orientation sensiblement inverse à celle d'un flux principal.

8. Pompe selon une des revendications précédentes, ***caractérisée en ce que,*** en cours de fonctionnement, un flux de base est alimenté par un écoulement venant de la roue à aube, l'orifice d'alimentation étant de préférence disposé plus loin radialement à l'extérieur que le point atteint par la roue à aube.

9. Pompe selon une des revendications précédentes, ***caractérisée en ce que,*** en cours de fonctionnement de la pompe, la roue à aube étant entraînée, un palier radial exerce par mécanisme d'écoulement une force de centrage sur la roue à aube dès que celle-ci est déviée.

10. Pompe selon une des revendications précédentes, ***caractérisée en ce qu'**un* palier axial est un palier à calotte sphérique ou un autre palier basculant.
